# EUROPEAN PATENT SPECIFICATION

(11) **EP 2 094 290 B1**
(45) Date of publication and mention of the grant of the patent: **19.07.2017**
(21) Application number: 07817959.5
(22) Date of filing: 02.11.2007
(51) Int. Cl.: A61K 38/19

(54) **ENHANCING PULMONARY HOST DEFENSE VIA ADMINISTRATION OF GRANULOCYTE-MACROPHAGE COLONY STIMULATING FACTOR**
VERSTÄRKUNG DER PULMONALEN WIRTSABWEHR DURCH VERABREICHUNG VON GRANULOZYTEN-MAKROPHAGEN-KOLONIESTIMULIERENDEM FAKTOR
RENFORCEMENT DES DÉFENSES PULMONAIRES ENDOGÈNES PAR ADMINISTRATION DE FACTEUR DE STIMULATION DE COLONIES GRANULOCYTES-MACROPHAGES

(30) Priority: 03.11.2006 US 864190 P; 02.07.2007 US 947511 P
(43) Date of publication of application: 02.09.2009
(73) Proprietor: Drugrecure ApS, 2200 Copenhagen N. (DK)
(72) Inventor: FIALA, Kaare, 2000 Frederiksberg (DK)
(74) Representative: Høiberg P/S
(86) International application number: PCT/DK2007/050161
(87) International publication number: WO 2008/052567

(56) References cited:
- WO-A1-88/00832
- TAZAWA RYUSHI ET AL: "Granulocyte-macrophage colony-stimulating factor inhalation therapy for patients with idiopathic pulmonary alveolar proteinosis: a pilot study; and long-term treatment with aerosolized granulocyte-macrophage colony-stimulating factor: a case report." RESPIROLOGY (CARLTON, VIC.) JAN 2006, vol. 11 Suppl, January 2006 (2006-01), pages S61-S64, XP002504690 ISSN: 1323-7799
- WYLAM M E ET AL: "Aerosol granulocyte-macrophage colony-stimulating factor for pulmonary alveolar proteinosis" EUROPEAN RESPIRATORY JOURNAL, vol. 27, no. 3, March 2006 (2006-03), pages 585-593, XP002502875 ISSN: 0903-1936
- PRICE A ET AL: "Pulmonary alveolar proteinosis associated with anti-GM-CSF antibodies in a child: Successful treatment with inhaled GM-CSF" PEDIATRIC PULMONOLOGY 200604 US, vol. 41, no. 4, April 2006 (2006-04), pages 367-370, XP002502876 ISSN: 8755-6863 1099-0496
- ARAI T ET AL: "Serum neutralizing capacity of GM-CSF reflects disease severity in a patient with pulmonary alveolar proteinosis successfully treated with inhaled GM-CSF" RESPIRATORY MEDICINE, BAILLIERE TINDALL, LONDON, GB, vol. 98, no. 12, 1 December 2004 (2004-12-01), pages 1227-1230, XP004644838 ISSN: 0954-6111
- BONFIELD T L ET AL: "Anti-GM-CSF titer predicts response to GM-CSF therapy in pulmonary alveolar proteinosis" CLINICAL IMMUNOLOGY, ACADEMIC PRESS, US, vol. 105, no. 3, 1 January 2002 (2002-01-01), pages 342-350, XP003011650 ISSN: 1521-6616
- NIVEN R W ET AL: "THE PULMONARY ABSORPTION OF AEROSOLIZED AND INTRATRACHEALLY INSTILLED RHG-CSF AND MONOPEGYLATED RHG-CSF" PHARMACEUTICAL RESEARCH, NEW YORK, NY, US, vol. 12, no. 9, 1 January 1995 (1995-01-01), pages 1343-1349, XP001013525 ISSN: 0724-8741
- YAMAMOTO HAJIME ET AL: "A combination therapy of whole lung lavage and GM-CSF inhalation in pulmonary alveolar proteinosis." PEDIATRIC PULMONOLOGY AUG 2008, vol. 43, no. 8, August 2008 (2008-08), pages 828-830, XP002502877 ISSN: 1099-0496
- ROSE R M ET AL: "The effect of aerosolized recombinant human granulocyte macrophage colony-stimulating factor on lung leukocytes in nonhuman primates", THE AMERICAN REVIEW OF RESPIRATORY DISEASE, AMERICAN THORACIC SOCIETY, US, vol. 146, no. 5 Pt. 1, 1 November 1992 (1992-11-01), pages 1279-1286, XP009162592, ISSN: 0003-0805
- S. HEROLD ET AL: "Inhaled Granulocyte/Macrophage Colony-Stimulating Fctor as Treatment of Pneumonia-associated Acute Respiratory Distress Syndrome", AMERICAN JOURNAL OF RESPIRATORY AND CRITICAL CARE MEDICINE, vol. 189, no. 5, 1 March 2014 (2014-03-01) , pages 609-611,

### SEQUENCE LISTING

<110> Drugrecure AS
<120> Enhancing Pulmonary Host Defense via Administration of Granulocyte-Macrophage Colony stimulating factor
<130> P1590 PC00
<160> 1
<170> PatentIn version 3.4
<210> 1
   <211> 144
   <212> PRT
   <213> Homo Sapiens
<400> 1
   Met Trp Leu Gln Ser Leu Leu Leu Leu Gly Thr Val Ala Cys Ser Ile
   1               5                   10                  15
   Ser Ala Pro Ala Arg Ser Pro Ser Pro Ser Thr Gln Pro Trp Glu His
               20                  25                  30
   Val Asn Ala Ile Gln Glu Ala Arg Arg Leu Leu Asn Leu Ser Arg Asp
           35                  40                  45
   Thr Ala Ala Glu Met Asn Glu Thr Val Glu Val Ile Ser Glu Met Phe
       50                  55                  60
   Asp Leu Gln Glu Pro Thr Cys Leu Gln Thr Arg Leu Glu Leu Tyr Lys
   65                  70                  75                  80
   Gln Gly Leu Arg Gly Ser Leu Thr Lys Leu Lys Gly Pro Leu Thr Met
                   85                  90                  95
   Met Ala Ser His Tyr Lys Gln His Cys Pro Pro Thr Pro Glu Thr Ser
               100                 105                 110
   Cys Ala Thr Gln Ile Ile Thr Phe Glu Ser Phe Lys Glu Asn Leu Lys
           115                 120                 125
   Asp Phe Leu Leu Val Ile Pro Phe Asp Cys Trp Glu Pro Val Gln Glu
       130                 135                 140

## Description

### Technical field

The present invention provides a method for enhancing pulmonary host defense in a subject suffering from, for example, but not limited to, lung cancer, pneumonia, pneumocystis carinii or cystic fibrosis and or critically ill patients with bacterial, fungal and/or viral infection and/or bacterial, fungal and/or viral colonization by administering to the subject an effective amount of granulocyte-macrophage colony stimulating factor (GM-CSF) via pulmonary airway administration.

### Background

Granulocyte-macrophage colony stimulating factor, GM-CSF, was originally identified as a hemopoietic growth factor. Human GM-CSF stimulates the growth of myeloid and erythroid progenitors in vitro and activates monocytes, macrophages and granulocytes in several immune and inflammatory processes (Gasson et al., 1990b; Gasson et al., 1990a; Hart et al., 1988; Rapoport et al., 1992). It is produced by a number of cell types including lymphocytes, monocytes, endothelial cells, fibroblasts and some malignant cells (Metcalf et al., 1986; Clark and Kamen, 1987; Hart et al, 1988; Metcalf et al., 1986). In addition to having a function of growth stimulation and differentiation on hemopoietic precursor cells, GM-CSF also was discovered as having a variety of effects on cells of the immune system expressing the GM-CSF receptor (for review see: Hamilton, 2002; de Groot et al., 1998).

Granulocyte-macrophage colony stimulating factor inhalation therapy has been disclosed for treating patients suffering from idiopathic pulmonary alveolar proteinosis, a rare lung disease characterized by the accumulation of surfactant that fills the terminal airways and alveoli, thereby impairing respiratory function (Tazawa et al. Respirology 2006 11:S61-S64).

Published U.S. Application No. 2004024762 discloses kits for the preparation of liquid compositions which can be administered to humans as aerosols for the diagnosis, prevention and treatment of human diseases. Included in the list of compounds which can be provided in the kits is granulocyte-macrophage colony stimulating factor.

Included in a list of diseases or condition suggested to be treated with these kits are cystic fibrosis and pneumonia.

An article by Rose et al. (Am Rev Respir Dis. 1992 Nov;146(5 Pt 1):1279-86) discloses a study of the administration of aerosolized GM-CSF to healthy cynomolgus monkeys. The article does not disclose treatment of diseases associated with infections of the pulmonary system.

WO88/00832 discloses the use of GM-CSF for treating infectious diseases, but does not disclose the pulmonary administration of GM-CSF for the treatment of pulmonary diseases associated with bacterial, viral and/or fungal infections.

### Summary of invention

In one aspect, the present disclosure relates to a granulocyte-macrophage colony stimulating factor (GM-CSF), or a functional homologue thereof having at least 75% sequence identity to human GM-CSF for use in treatment of pulmonary diseases associated with bacterial, fungal and/or viral infection or colonization of the pulmonary system, wherein said GM-CSF, or a functional homologue thereof, is to be administered via pulmonary administration.

The GM-CSF of the present disclosure is particularly useful in alleviating symptoms and/or treating pulmonary diseases including, but not limited to lung cancer, pneumocystis carinii, pneumonia with or without bacterial, fungal and/or viral infection including but not limited to community acquired pneumonia, nosocomial pneumonia or ventilator associated pneumonia, and or cystic fibrosis with or without bacterial, fungal and/or viral infection or colonization, bronchitis, Bronchiectasis, Diffuse panbronchiolitis, Bronchiolitis, Bronchiolitis obliterans, Bronchiolitis obliterans organizing pneumonia (BOOP) with or without bacterial, fungal and/or viral colonization and/or bacterial, fungal and/or viral infection or colonization.

Another aspect of the present disclosure relates to the use of GM-CSF or a functional homologue thereof having at least 75% sequence identity to human GM-CSF for the manufacture of a medicament for pulmonary administration for use in the treatment of pulmonary diseases associated with bacterial, fungal and/or viral infection or colonization of the pulmonary system. GM-CSF via pulmonary administration is particularly useful in treating pulmonary diseases and/or alleviating symptoms including, but not limited to lung cancer, pneumonia, pneumocystis carinii and cystic fibrosis with bacterial, fungal and/or viral infection and/or bacterial, fungal and/or viral colonization.

### Detailed description

The present invention is as defined in the claims.

The present disclosure relates to the intratracheal, intrabronchial or bronchio-alveolar administration, by any appropriate method including, but not limited to, intratracheal, intrabronchial or intraalveolar administration, to a human subject inclusive of both adults and children, of purified or concentrated natural human of granulocyte-macrophage colony stimulating factor (GM-CSF), or a functional homologue of thereof, however prepared, to enhance the pulmonary host defense.

Administration of an effective amount of GM-CSF or a functional homologue of thereof via intratracheal, intrabronchial or bronchio-alveolar administration is particularly useful in alleviating symptoms and/or treating subjects suffering from pulmonary conditions including, but not limited to lung cancer, both small cell lung cancer and squamous or non-small cell lung cancer, pneumonia, pneumocystis carinii and cystic fibrosis with bacterial, fungal such as, but not limited to, *Candidiasis* species and *Aspergillus* species or other less common mycostic strains, and/or viral infection and/or bacterial, fungal such as, but not limited to, *Candidiasis* species and *Aspergillus* species, and/or viral colonization of the airways and/or lung parenchyma.

### GM-CSF

Colony-stimulating factors are glycoproteins that stimulate the growth of hematopoietic progenitors and enhance the functional activity of mature effector cells. In brief, at the level of immature cells, CSF's assure the self-renewal of the staminal pool and activate the first stage of hematopoietic differentiation; in the middle stage, when cell proliferation is associated to a progressive acquisition of characteristics of mature cells, they enormously enhance the number of differentiating cells; in the terminal stage they control the circulation and the activation of mature cells.

Mature GM-CSF is a monomeric protein of 127 amino acids with several potential glycosylation sites. The variable degree of glycosylation results in a molecular weight range between 14kDa and 35kDa. Non-glycosylated and glycosylated GM-CSF show similar activity in vitro (Cebon et al., 1990). The crystallographic analysis of GM-CSF revealed a barrel- shaped structure composed of four short alpha helices (Diederichs et al., 1991). There are two known sequence variants of GM-CSF.

GM-CSF exerts its biological activity by binding to its receptor. The most important sites of GM-CSF receptor (GM-CSF-R) expression are on the cell surface of myeloid cells, like alveolar macrophages type I & II, epithelial pulmonary cells and endothelial cells, whereas lymphocytes are GM-CSF-R negative. The native receptor is composed of at least two subunits, alpha and beta. The alpha subunit imparts ligand specificity and binds GM-CSF with nanomolar affinity (Gearing et al., 1989; Gasson et al., 1986). The beta subunit is also part of the interleukin-3 and interleukin-5 receptor complexes and, in association with the GM-CSF receptor alpha subunit and GM-CSF, leads to the formation of a complex with picomolar binding affinity (Hayashida et al., 1990). The binding domains on GM-CSF for the receptor have been mapped: GM-CSF interacts with the beta subunit of its receptor via a very restricted region in the first alpha helix of GM-CSF (Shanafelt et al., 1991 b; Shanafelt et al., 1991 a; Lopez et al., 1991). Binding to the alpha subunit could be mapped to the third alpha helix, helix C, the initial residues of the loop joining helices C and D, and to the carboxyterminal tail of GM-CSF (Brown et al., 1994).

Formation of the GM-CSF trimeric receptor complex leads to the activation of complex signaling cascades involving molecules of the JAK/STAT families, She, Ras, Raf, the MAP kinases, phosphatidylinositol-3 -kinase and NFkB, finally leading to transcription of c-myc, c-fos and c-jun. Activation is mainly induced by the beta subunit of the receptor (Hayashida et al., 1990; Kitamura et al., 1991; Sato et al., 1993). The shared beta subunit is also responsible for the overlapping functions exerted by IL-3, IL-5 and GM- CSF (for review see: de Groot et al., 1998).

Apart from its hemopoietic growth and differentiation stimulating activity, GM-CSF functions especially as a proinflammatory cytokine. Macrophages, e.g. alveolar macrophages type I & II and monocytes as well as neutrophils and eosinophils become activated by GM-CSF, resulting in the release of other cytokines and chemokines, matrix degrading proteases, increased HLA expression and increased expression of cell adhesion molecules or receptors for CC-chemokinesm which in turn, leads to increased chemotaxis of inflammatory cells into inflamed tissue.

Wong et al., Science Vol. 228, pp. 810-815 (1985) and Kaushansky et al., Proc. Natl. Acad. Sci. USA, Vol. 83, pp. 3101-3105 (1986) have described the production of recombinant GM-CSF in mammalian cells. Burgess et al., Blood, Vol. 69, pp. 43-51 (1987) describes the purification of GM-CSF produced in Escherichia coli.

### Functional homologues

A functional homologue of GM-CSF is a polypeptide having at least 50 % sequence identity with SEQ ID NO. 1 and has one or more GM-CSF functions, such as the stimulation of the growth and differentiation of hematopoietic precursor cells from various lineages, including granulocytes, macrophages, eosinophils and erythrocytes.

GM-CSF regulates multiple functions of alveolar macrophages (AM). GM-CSF stimulation of AM has been documented to enhance alveolar macrophages selectively respond to noxious ingestants, i.e., stimulation of inflammation during bacterial phagocytosis, nonnoxious ingestants are generally mollified, i.e., antiinflammatory responses during phagocytosis of apoptotic cells. Further AM functions are enhanced by GM-CSF stimulation with subsequent proliferation, differentiation, accumulation and activation. Further these GM-CSF effects also encompasses cell adhesion, improved chemotaxis, Fc-receptor expression, complement- and antibody-mediated phagocytosis, oxidative metabolism, intracellular killing of bacteria, fungi, protozoa, and viruses, cytokine signaling, and antigen presentation. Further GM-CSF enhances defects in AM cell adhesion, pathogen associated molecular pattern receptors, like Toll-like receptors and TLR trans-membranous signaling, surfactant protein and lipid uptake and degradation (Trapnell BC and Whitsett JA. GM-CSF regulates pulmonary surfactant homeostasis and alveolar macrophage-mediated innate host defense. Annu. Rev. Physiol. 2002.64:775-802).

Further GM-CSF interacts with the AM's recognition receptors, the so-called toll like receptors (TLR). GM-CSF is important in the pulmonary host defense in pneumonia due to its interaction with the TLR's participation in the host defense resulting in enhanced clearance of the causative microorganism (Chen GH, Olszewski MA, McDonald RA, Wells JC, Paine R 3rd, Huffnagle GB, Toews GB.Role of granulocyte macrophage colony-stimulating factor in host defense against pulmonary Cryptococcus neoformans infection during murine allergic bronchopulmonary mycosis. Am J Pathol. 2007 Mar;170(3):1028-40). Lung has its own innate GM-CSF production, which is reduced in pneumonia and hyperoxia, in relation to high O₂ exposure as seen in, e.g. ventilator associated pneumonia (VAP) contributing impairment of host defense secondary to apoptosis with poor response to infections. The hyperoxic injury seems to be counteracted by activation of alveolar macrophages with GM-CSF (Altemeier WA, Sinclair SE. Hyperoxia in the intensive care unit: why more is not always better. Curr Opin Crit Care. 2007 Feb;13(1):73-8. & Baleeiro CE, Christensen PJ, Morris SB, Mendez MP, Wilcoxen SE, Paine R.GM-CSF and the impaired pulmonary innate immune response following hyperoxic stress. Am J Physiol Lung Cell Mol Physiol. 2006 Dec;291(6):L1246-55. Epub 2006 Aug 4) with subsequent clearance of P. aeruginosa via expression of the TLR signaling pathway (Baleeiro CE, Christensen PJ, Morris SB, Mendez MP, Wilcoxen SE, Paine R.GM-CSF and the impaired pulmonary innate immune response following hyperoxic stress. Am J Physiol Lung Cell Mol Physiol. 2006 Dec;291(6):L1246-55. Epub 2006 Aug 4).

Finally GM -CSF produces in-vitro conversion of AM into immature dendritic cells (DC), which may further be matured with specific agents in respect to activate the homing of matured DC's to a specified receptor or target. (Zobywalski A, Javorovic M, Frankenberger B, Pohla H, Kremmer E, Bigalke I, Schendel DJ. Generation of clinical grade dendritic cells with capacity to produce biologically active IL-12p70. J Transl Med. 2007 Apr 12;5:18).

Preferably, evolutionary conservation between GM-CSF of different closely related species, e.g. assessed by sequence alignment, can be used to pinpoint the degree of evolutionary pressure on individual residues. Preferably, GM-CSF sequences are compared between species where GM-CSF function is conserved, for example but not limited to mammals including rodents, monkeys and apes. Residues under high selective pressure are more likely to represent essential amino acids that cannot easily be substituted than residues that change between species. It is evident from the above that a reasonable number of modifications or alterations of the human GM-CSF sequence does not interfere with the activity of the GM-CSF molecule according to the present disclosure. Such GM-CSF molecules are herein referred to as functional equivalents of human GM-CSF, and may be such as variants and fragments of native human GM-CSF as described here below.

As used herein the expression "variant" refers to polypeptides or proteins which are homologous to the basic protein, which is suitably human GM-CSF, but which differs from the base sequence from which they are derived in that one or more amino acids within the sequence are substituted for other amino acids. Amino acid substitutions may be regarded as "conservative" where an amino acid is replaced with a different amino acid with broadly similar properties. Non-conservative substitutions are where amino acids are replaced with amino acids of a different type. Broadly speaking, fewer non-conservative substitutions will be possible without altering the biological activity of the polypeptide.

A person skilled in the art will know how to make and assess 'conservative' amino acid substitutions, by which one amino acid is substituted for another with one or more shared chemical and/or physical characteristics. Conservative amino acid substitutions are less likely to affect the functionality of the protein. Amino acids may be grouped according to shared characteristics. A conservative amino acid substitution is a substitution of one amino acid within a predetermined group of amino acids for another amino acid within the same group, wherein the amino acids within a predetermined groups exhibit similar or substantially similar characteristics. Within the meaning of the term "conservative amino acid substitution" as applied herein, one amino acid may be substituted for another within groups of amino acids characterised by having
i) polar side chains (Asp, Glu, Lys, Arg, His, Asn, Gln, Ser, Thr, Tyr, and Cys,)
ii) non-polar side chains (Gly, Ala, Val, Leu, Ile, Phe, Trp, Pro, and Met)
iii) aliphatic side chains (Gly, Ala Val, Leu, Ile)
iv) cyclic side chains (Phe, Tyr, Trp, His, Pro)
v) aromatic side chains (Phe, Tyr, Trp)
vi) acidic side chains (Asp, Glu)
vii) basic side chains (Lys, Arg, His)
viii) amide side chains (Asn, Gin)
ix) hydroxy side chains (Ser, Thr)
x) sulphor-containing side chains (Cys, Met), and
xi) amino acids being monoamino-dicarboxylic acids or monoaminomonocarboxylic-monoamidocarboxylic acids (Asp, Glu, Asn, Gin).

A functional homologue within the scope of the present disclosure is a polypeptide that exhibits at least 50% sequence identity with human GM-CSF as identified by SEQ ID NO. 1, preferably at least 60%, 70% sequence identity preferably functional homologues have at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with SEQ ID NO: 1.

Sequence identity can be calculated using a number of well-known algorithms and applying a number of different gap penalties. Any sequence alignment algorithm, such as but not limited to FASTA, BLAST, or GETSEQ may be used for searching homologues and calculating sequence identity. Moreover, when appropriate any commonly known substitution matrix, such as but not limited to PAM, BLOSSUM or PSSM matrices, may be applied with the search algorithm. For example, a PSSM (position specific scoring matrix) may be applied via the PSI-BLAST program.

Moreover, sequence alignments may be performed using a range of penalties for gap opening and extension. For example, the BLAST algorithm may be used with a gap opening penalty in the range 5-12, and a gap extension penalty in the range 1-2.

Accordingly, a variant or a fragment thereof according to the present disclosure may comprise, within the same variant of the sequence or fragments thereof, or among different variants of the sequence or fragments thereof, at least one substitution, such as a plurality of substitutions introduced independently of one another.

It is clear from the above outline that the same variant or fragment thereof may comprise more than one conservative amino acid substitution from more than one group of conservative amino acids as defined herein above.

Aside from the twenty standard amino acids and two special amino acids, selenocysteine and pyrrolysine, there are a vast number of "nonstandard amino acids" which are not incorporated into protein in vivo. Examples of nonstandard amino acids include the sulfur-containing taurine and the neurotransmitters GABA and dopamine. Other examples are lanthionine, 2-Aminoisobutyric acid, and dehydroalanine. Further non standard amino are ornithine and citrulline.

Non-standard amino acids are usually formed through modifications to standard amino acids. For example, taurine can be formed by the decarboxylation of cysteine, while dopamine is synthesized from tyrosine and hydroxyproline is made by a posttranslational modification of proline (common in collagen). Examples of non-natural amino acids are those listed e.g. in 37 C.F.R. section 1.822(b)(4).
Both standard and non standard amino acid residues described herein can be in the "D" or or "L" isomeric form.

It is contemplated that a functional equivalent according to the present disclosure may comprise any amino acid including non-standard amino acids. In preferred embodiments according to the present disclosure a functional equivalent comprises only standard amino acids.

The standard and/or non-standard amino acids may be linked by peptide bonds or by non-peptide bonds. The term peptide also embraces post-translational modifications introduced by chemical or enzyme-catalyzed reactions, as are known in the art. Such post-translational modifications can be introduced prior to partitioning, if desired. Amino acids as specified herein will preferentially be in the L-stereoisomeric form. Amino acid analogs can be employed instead of the 20 naturally-occurring amino acids. Several such analogs are known, including fluorophenylalanine, norleucine, azetidine-2-carboxylic acid, S-aminoethyl cysteine, 4-methyl tryptophan and the like.

Suitably variants will be at least 60% identical, preferably at least 70% and accordingly, variants preferably have at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with the predetermined sequence of human GM-CSF.

Functional equivalents may further comprise chemical modifications such as ubiquitination, labeling (e.g., with radionuclides, various enzymes, etc.), pegylation (derivatization with polyethylene glycol), or by insertion (or substitution by chemical synthesis) of amino acids (amino acids) such as ornithine, which do not normally occur in human proteins.

In addition to the peptidyl compounds described herein, sterically similar compounds may be formulated to mimic the key portions of the peptide structure and that such compounds may also be used in the same manner as the peptides of the present disclosure. This may be achieved by techniques of modelling and chemical designing known to those of skill in the art. For example, esterification and other alkylations may be em-ployed to modify the amino terminus of, e.g., a di-arginine peptide backbone, to mimic a tetra peptide structure. It will be understood that all such sterically similar constructs fall within the scope of the present disclosure.

Peptides with N-terminal alkylations and C-terminal esterifications are also encompassed within the present disclosure. Functional equivalents also comprise glycosylated and covalent or aggregative conjugates formed with the same molecules, including dimers or unrelated chemical moieties. Such functional equivalents are prepared by linkage of functionalities to groups which are found in fragment including at any one or both of the N- and C-termini, by means known in the art.

The term "fragment thereof" may refer to any portion of the given amino acid sequence. Fragments may comprise more than one portion from within the full-length protein, joined together. Suitable fragments may be deletion or addition mutants. The addition of at least one amino acid may be an addition of from preferably 2 to 250 amino acids, such as from 10 to 20 amino acids, for example from 20 to 30 amino acids, such as from 40 to 50 amino acids. Fragments may include small regions from the protein or combinations of these.

Suitable fragments may be deletion or addition mutants. The addition or deletion of at least one amino acid may be an addition or deletion of from preferably 2 to 250 amino acids, such as from 10 to 20 amino acids, for example from 20 to 30 amino acids, such as from 40 to 50 amino acids. The deletion and/or the addition may - independently of one another - be a deletion and/or an addition within a sequence and/or at the end of a sequence.

Deletion mutants suitably comprise at least 20 or 40 consecutive amino acid and more preferably at least 80 or 100 consecutive amino acids in length. Accordingly such a fragment may be a shorter sequence of the sequence as identified by SEQ ID NO: 1 comprising at least 20 consecutive amino acids, for example at least 30 consecutive amino acids, such as at least 40 consecutive amino acids, for example at least 50 consecutive amino acids, such as at least 60 consecutive amino acids, for example at least 70 consecutive amino acids, such as at least 80 consecutive amino acids, for example at least 90 consecutive amino acids, such as at least 95 consecutive amino acids, such as at least 100 consecutive amino acids, such as at least 105 amino acids, for example at least 110 consecutive amino acids, such as at least 115 consecutive amino acids, for example at least 120 consecutive amino acids, wherein said deletion mutants preferably has at least 75% sequence identity, for example at least 80% sequence identity, such as at least 85 % sequence identity, for example at least 90 % sequence identity, such as at least 91 % sequence identity, for example at least 91% sequence identity, such as at least 92 % sequence identity, for example at least 93 % sequence identity, such as at least 94 % sequence identity, for example at least 95 % sequence identity, such as at least 96 % sequence identity, for example at least 97% sequence identity, such as at least 98 % sequence identity, for example 99% sequence identity with SEQ ID NO: 1.

It is preferred that functional homologues of GM-CSF comprises at the most 500, more preferably at the most 400, even more preferably at the most 300, yet more preferably at the most 200, such as at the most 175, for example at the most 160, such as at the most 150 amino acids, for example at the most 144 amino acids.

The term "fragment thereof" may refer to any portion of the given amino acid sequence. Fragments may comprise more than one portion from within the full-length protein, joined together. Portions will suitably comprise at least 5 and preferably at least 10 consecutive amino acids from the basic sequence. They may include small regions from the protein or combinations of these.

There are two known variants of human GM-CSF; a T115I substitution in variant and a I117T substitution in variant 2. Accordingly, in one embodiment of the present disclosure functional homologues of GM-CSF comprises a sequence with high sequence identity to SEQ ID NO: 1 or any of the splice variants.

Analogs of GM-CSF are for example described in U.S. Pat. Nos. 5,229,496, 5,393,870, and 5,391,485 to Deeley, et al. Such analogues are also functional equivalents comprised within the present disclosure.

### Recombinant production

The present disclosure relates to the pulmonary administration, of granulocyte-macrophage colony stimulating factor (GM-CSF), or a functional homologue of thereof, however prepared, to enhance the pulmonary host defense. GM-CSF can be produced in various ways, such as isolation from for example human or animal serum or from expression in cells, such as prokaryotic cells, yeast cells, insect cells, mammalian cells or in cell-free systems.

In one embodiment of the present disclosure, GM-CSF is produced recombinantly by host cells.

Thus, in one aspect of the present disclosure, GM-CSF is produced by host cells comprising a first nucleic acid sequence encoding the GM-CSF operably associated with a second nucleic acid capable of directing expression in said host cells. The second nucleic acid sequence may thus comprise or even consist of a promoter that will direct the expression of protein of interest in said cells. A skilled person will be readily capable of identifying useful second nucleic acid sequence for use in a given host cell.

The process of producing recombinant GM-CSF in general comprises the steps of:
- providing a host cell
- preparing a gene expression construct comprising a first nucleic acid encoding GM-CSF operably linked to a second nucleic acid capble of directing expression of said protein of interest in the host cell
- transforming the host cell with the construct,
- cultivating the host cell, thereby obtaining expression of GM-CSF.

The recombinant GM-CSF thus produced may be isolated by any conventional method, such as any of the methods for protein isolation described herein below. The skilled person will be able to identify a suitable protein isolation steps for purifying GM-CSF.

In one embodiment of the present disclosure, the recombinantly produced GM-CSF is excreted by the host cells. When GM-CSF is excreted the process of producing a recombinant protein of interest may comprise the following steps
- providing a host cell
- preparing a gene expression construct comprising a first nucleic acid encoding GM-CSF operably linked to a second nucleic acid capable of directing expression of said protein of interest in said host cell
- transforming said host cell with the construct,
- cultivating the host cell, thereby obtaining expression of GM-CSF and secretion of GM-CSF into the culture medium,
- thereby obtaining culture medium comprising GM-CSF.

The composition comprising GM-CSF and nucleic acids may thus in this embodiment of the present disclosure be the culture medium or a composition prepared from the culture medium.

In another embodiment of the present disclosure said composition is an extract prepared from animals, parts thereof or cells or an isolated fraction of such an extract.

In an embodiment of the present disclosure, GM-CSF is recombinantly produced in vitro in host cells and is isolated from cell lysate, cell extract or from tissue culture supernatant. In a more preferred embodiment according to the present disclosure GM-CSF is produced by host cells that are modified in such a way that they express GM-CSF. In an even more preferred embodiment of the present disclosure said host cells are transformed to produce and excrete GM-CSF.

### The pulmonary host defense

The respiratory system is the first line of defense against inhaled substances. The system comprises a complex and multilayered defense system involving mechanical, reflex, cellular mechanisms as well as producing local and systemically derived defense molecules.

The upper airways and the major bronchi are protecting the lungs with the anatomic barriers they represent associated with the cough reflex,the mucociliary apparatus and the secretory immunoglobulin A (IgA). Below, the superficial layers of the mucosa, a tight network of dendritic cells will sense and catch any invading organisms and bring them to the lymph nodes around the airways or in the hilum. In the respiratory units beyond the respiratory bronchioles particles will be caught by alveolar macrophages in a milieu rich in elements such as IgG, complement, surfactant and fibronectin. In these units when needed various amounts of neutrophils and lymphocytes will be recruited.

The pulmonary epithelium in particular protects the airspace and preserves normal respiratory functions by providing a barrier function and by secreting substances which targets environmental challenges. The pulmonary epithelium can be injured by infection, such as infections by viruses, which permits bacterial attachment.

The cellular part of the pulmonary defense involves mucins, antibiotic substances and/or antioxidants such as surfactants, immunoglobulins and complement proteins and antiproteases. Collectins have also been implicated in the cellular pulmonary defense.

Inflammatory cells are recruited to the airways via chemoattractants secreted by the pulmonary epithelium, which in turn leads to the migration of inflammatory cells across the epithelium. The epithelium further releases cytokines to regulate the inflammatory cell activities. The epithelial cells release factors such as IL-8 and GM-CSF, whereas macrophages release IL-1 and TNF, which further stimulates the epithelial cells.

For purposes of the present disclosure by "enhancing pulmonary host defense" it is meant any detectable change in the host which increase defense to infection in the pulmonary system of the host. Enhancement of the pulmonary host defense can be determined, for example, by monitoring local pulmonary host defense parameters such as changes in white cell count and/or cytokine release from tracheal spirate or obtained from bronchoalveolar lavage. Alternatively or in addition, enhancement of the pulmonary host defense can be determined by flow cytometric analysis of cells from the lung such as alveolar macrophages with or without selected and/or specified surface receptors and/or subgroups recruited by, for example, tracheal aspirate and/or from bronchoalveolar lavage fluid. Methods for flow cytometric analysis of cells from the lung are described Garn et al. in Experimental and Toxicologic Pathology 2006 57:S2:21-24.

### Medical indications

Administration of an effective amount of GM-CSF or a functional homologue of thereof via intratracheal, intrabronchial or bronchio-alveolar administration is particularly useful in alleviating symptoms and/or treating subjects suffering from conditions including, but not limited to lung cancer, both small cell lung cancer and squamous or non-small cell lung cancer, pneumonia, pneumocystis carinii and cystic fibrosis with bacterial, fungal or other less common mycostic strains, and/or viral infection and/or bacterial, fungal and/or viral colonization of the airways and/or lung parenchyma. The spectrum of diseases encompasses the following pulmonary conditions and or infections like Bronchitis, Cystic fibrosis, Bronchiectasis, Diffuse panbronchiolitis, Bronchiolitis, Bronchiolitis obliterans, Bronchiolitis obliterans organizing pneumonia (BOOP), Pneumonia of any cause, including but not limited to Community acquired pneumonia, Nosocomial pneumonia and Ventilator associated pneumonia (VAP).

Infections may for example be an infection by bacteria, fungi, viruses, parasites. For example infection by parasites such as plasmodium falciparum. For example infection by one or more bacteria selected from the group consisting of Achromobacter xylosoxidans, Acinetobacter calcoaceticus, preferably A. anitratus, A. haemolyticus, A. alcaligenes, and A. Iwoffii, Actinomyces israelii, Aeromonas hydrophilia, Alcaligenes species, preferably A. faecalis, A. odorans and A. denitrificans, Arizona hinshawii, Bacillus anthracis, Bacillus cereus, Bacteroides fragilis, Bacteroides melaninogenicus, Bordetella pertussis, Borrelia burgdorferi, Borrelia recurrentis, Brucella species, preferably B. abortus, B. suis, B. melitensis and B. canis, Calymmatobacterium granulomatis, Campylobacter fetus ssp. intestinalis, Campylobacter fetus ssp. jejuni, Chlamydia species, preferably C. psittaci and C. trachomatis, Chromobacterium violaceum, Citrobacter species, preferably C. freundii and C. diversus, Clostridium botulinum, Clostridium perfringens, Clostridium difficile, Clostridium tetani, Corynebacterium diphtheriae, Corynebacterium, preferably C. ulcerans, C. haemolyticum and C. pseudotuberculosis, Coxiella burnetii, Edwardsiella tarda, Eikenella corrodens, Enterobacter, preferably E. cloacae, E. aerogenes, E. hafniae (also named Hafnia alvei) and E. agglomerans, Erysipelothrix rhusiopathiae, Escherichia coli, Flavobacterium meningosepticum, Francisella tularensis, Fusobacterium nucleatum, Gardnerella vaginalis, Haemophilus ducreyi, Haemophilus influenzae, Helicobacter species, Klebsiella species, preferably K. pneumoniae, K. ozaenae og K. rhinoscleromatis, Legionella species, Leptospira interrogans, Listeria monocytogenes, Moraxella species, preferably M. lacunata and M. osloensis, Mycobacterioum bovis, Mycobacterium leprae, Mycobacterium tuberculosis, Mycoplasma species, preferably M. pneumoniae, Neisseria gonorrhoeae, Neisseria meningitidis, Nocardia species, preferably N. asteroides and N. brasiliensis, Pasterurella haemolytica, Pasteurella multocida, Peptococcus magnus, Plesiomonas shigelloides, Pneumococci, Proteus species, preferably P. mirabilis, P. vulgaris, P. rettgeri and P. morganii (also named Providencia rettgeri and Morganella morganii respectively), Providencia species, preferably P. alcalifaciens, P. stuartii and P. rettgeri (also named Proteus rettgeri), Pseudomonas aeruginosa, Pseudomonas mallei, Pseudomonas pseudomallei, Rickettsia, Rochalimaia henselae, Salmonella species, preferably S. enteridis, S. typhi and S. derby, and most preferably Salmonella species of the type Salmonella DT104, Serratia species, preferably S. marcescens, Shigella dysenteriae, S. flexneri, S. boydii and S. sonnei, Spirillum minor, Staphylococcus aureus, Staphylococcus epidermidis, Staphylococcus saprophyticus, Streptobacillus moniliformis, Streptococcus, preferably S. faecalis, S. faecium and S. durans, Streptococcus agalactiae, Streptococcus pneumoniae, Streptococcus pyogenes, Treponema carateum, Treponeam pallidum, Treponema pertenue, preferably T. pallidum, Ureaplasma urealyticum, Vibrio cholerae, Vibrio parahaemolyticus, Yersinia enterocolitica, and Yersinia pestis.

Infections also comprise protozoan infections such as, but not limited to, Trichomonas infections, such as Pentatrichomonas infections. For example T. buccalis, T. tenax, T. foetus, T. galli'nae, T. gallina'rum, T. ho'minis, T. intestinalis, T. te'nax, T. vaginalis.

In other embodiments of the present disclosure GM-CSF may be used for the treatment of any condition caused by fungal infections including, but not limited to: Aspergillosis, Blastomycosis, Candidiasis, Coccidioidomycosis, Cryptococcosis, Histoplasmosis, Paracoccidiomycosis, Sporotrichosis, Zygomycosis, pneumocystis carinii. The composition may also be used to treat fungal infections in conditions such as Chromoblastomycosis, Mycotic keratitis, Endogenous oculomycosis, Extension oculomycosis, Lobomycosis, Mycetoma, Nail, Hair, and Skin diseases (for example Onychomycosis (Tinea unguium), Piedra, Pityriasis versicolor, Tinea barbae, Tinea capitis, Tinea corporis, Tinea cruris, Tinea favosa, Tinea nigra, Tinea pedis), Otomycosis, Phaeohyphomycosis, Rhinosporidiosis.

### Administration

Methods of intratracheal, intrabronchial or bronchio-alveolar administration include, but are not limited to, spraying, lavage, inhalation, flushing or installation, using as fluid a physiologically acceptable composition in which GM-CSF have been dissolved. When used herein the terms "intratracheal, intrabronchial or intraalveolar administration" include all forms of such administration whereby GM-CSF is applied into the trachea, the bronchi or the alveoli, respectively, whether by the instillation of a solution of GM-CSF, by applying GM-CSF in a powder form, or by allowing GM-CSF to reach the relevant part of the airway by inhalation of GM-CSF as an aerosolized or nebulized solution or suspension or inhaled powder or gel, with or without added stabilizers or other excipients.

Methods of intrabronchial/alveolar administration include, but are not limited to, bronchoalveolar lavage (BAL) according to methods well known to those skilled in the art, using as a lavage fluid a physiologically acceptable composition in which GM-CSF been dissolved or indeed by any other effective form of intrabronchial administration including the use of inhaled powders containing GM-CSF in dry form, with or without excipients, or the direct application of GM-CSF, in solution or suspension or powder form during bronchoscopy. Methods for intratracheal administration include, but are not limited to, blind tracheal washing with a similar solution of dissolved GM-CSF or a GM-CSF suspension, or the inhalation of nebulized fluid droplets containing dissolved GM-CSF or a GM-CSF suspension obtained by use of any nebulizing apparatus adequate for this purpose.

In another embodiment according to the present disclosure, intratracheal, intrabronchial or intraalveolar administration does not include inhalation of the product but the instillation or application of a solution of GM-CSF or a powder or a gel containing GM-CSF into the trachea or lower airways.

Other preferred methods of administration may include using the following devices:
1. Pressurized nebulizers using compressed air/oxygen mixture
2. Ultrasonic nebulizers
3. Electronic micropump nebulizers (e.g. Aeroneb Professional Nebulizer)
4. Metered dose inhaler (MDI)
5. Dry powder inhaler systems (DPI),

The aerosol may be delivered by via a) facemasks or b) via endotracheal tubes in intubated patients during mechanical ventilation (device 1, 2 and 3). The devices 4 and 5 can also be used by the patient without assistance provided that the patient is able to self-activate the aerosol device.

Preferred concentrations for a solution comprising GM-CSF and/or functional homologues or variants of GM-CSF are in the range of 0.1 µg to 10000 µg active ingredient per ml solution. The suitable concentrations are often in the range of from 0.1 µg to 5000 µg per ml solution, such as in the range of from about 0.1 µg to 3000 µg per ml solution, and especially in the range of from about 0.1 µg to 1000 µg per ml solution, such as in the range of from about 0.1 µg to 250 µg per ml solution. A preferred concentration would be from about 0.1 to about 5.0 mg, preferably from about 0.3 mg to about 3.0 mg, such as from about 0.5 to about 1.5 mg and especially in the range from 0.8 to 1.0 mg per ml solution.

### Pharmaceutical composition

Pharmaceutical compositions or formulations for use in the present disclosure include GM-CSF or functional homologue thereof combination with, preferably dissolved in, a pharmaceutically acceptable carrier, preferably an aqueous carrier or diluent, or carried to the lower airways as a pegylated preparation or as a liposomal or nanoparticle preparation administered as an aerosol via inhalation, or as a lavage fluid administered via a bronchoscope as a bronchoalveloar lavage or as a blind intratracheal wash or lavage. A variety of aqueous carriers may be used, including, but not limited to 0.9% saline, buffered saline, physiologically compatible buffers and the like. The compositions may be sterilized by conventional techniques well known to those skilled in the art. The resulting aqueous solutions may be packaged for use or filtered under aseptic conditions and freeze-dried, the freeze-dried preparation being dissolved in a sterile aqueous solution prior to administration

In one embodiment according to the present disclosure a freeze-dried GM-CSF preparation may be pre-packaged for example in single dose units. In an even more preferred embodiment according to the present disclosure the single dose unit is adjusted to the patient.

The compositions may contain pharmaceutically acceptable auxiliary substances or adjuvants, including, without limitation, pH adjusting and buffering agents and/or tonicity adjusting agents, such as, for example, sodium acetate, sodium lactate, sodium chloride, potassium chloride, calcium chloride, etc.

The formulations may contain pharmaceutically acceptable carriers and excipients including microspheres, liposomes, microcapsules, nanoparticles or the like. Conventional liposomes are typically composed of phospholipids (neutral or negatively charged) and/or cholesterol. The liposomes are vesicular structures based on lipid bilayers surrounding aqueous compartments. They can vary in their physiochemical properties such as size, lipid composition, surface charge and number and fluidity of the phospholipids bilayers. The most frequently used lipid for liposome formation are: 1,2-Dilauroyl-*sn*-Glycero-3-Phosphocholine (DLPC), 1,2-Dimyristoyl-*sn*-Glycero-3-Phosphocholine (DMPC), 1,2-Dipalmitoyl-*sn*-Glycero-3-Phosphocholine (DPPC), 1,2-Distearoyl-*sn*-Glycero-3-Phosphocholine (DSPC), 1,2-Dioleoyl-*sn*-Glycero-3-Phosphocholine (DOPC), 1,2-Dimyristoyl-*sn*-Glycero-3-Phosphoethanolamine (DMPE), 1,2-Dipalmitoyl-*sn*-Glycero-3-Phosphoethanolamine (DPPE), 1,2-Dioleoyl-*sn*-Glycero-3-Phosphoethanolamine (DOPE), 1,2-Dimyristoyl-*sn*-Glycero-3-Phosphate (Monosodium Salt) (DMPA), 1,2-Dipalmitoyl-*sn*-Glycero-3-Phosphate (Monosodium Salt) (DPPA), 1,2-Dioleoyl-*sn*-Glycero-3-Phosphate (Monosodium Salt) (DOPA), 1,2-Dimyristoyl-*sn*-Glycero-3-[Phospho-*rac*-(1-glycerol)] (Sodium Salt) (DMPG), 1,2-Dipalmitoyl-*sn*-Glycero-3-[Phospho-*rac*-(1-glycerol)] (Sodium Salt) (DPPG), 1,2-Dioleoyl-*sn*-Glycero-3-[Phospho-*rac*-(1-glycerol)] (Sodium Salt) (DOPG), 1,2-Dimyristoyl-*sn*-Glycero-3-[Phospho-L-Serine] (Sodium Salt) (DMPS), 1,2-Dipalmitoyl-*sn*-Glycero-3-[Phospho-L-Serine) (Sodium Salt) (DPPS), 1,2-Dioleoyl-*sn*-Glycero-3-[Phospho-L-Serine] (Sodium Salt) (DOPS), 1,2-Dioleoyl-*sn*-Glycero-3-Phosphoethanolamine-N-(glutaryl) (Sodium Salt) and 1,1',2,2'-Tetramyristoyl Cardiolipin (Ammonium Salt). Formulations composed of DPPC in combination with other lipids or modifiers of liposomes are preferred e.g. in combination with cholesterol and/or phosphatidylcholine.

Long-circulating liposomes are characterized by their ability to extravasate at body sites where the permeability of the vascular wall is increased. The most popular way of producing long-circulating liposomes is to attach hydrophilic polymer polyethylene glycol (PEG) covalently to the outer surface of the liposome. Some of the preferred lipids are: 1,2-Dipalmitoyl-*sn*-Glycero-3-Phosphoethanolamine-N-[Methoxy(Polyethylene glycol)-2000] (Ammonium Salt), 1,2-Dipalmitoyl-*sn*-Glycero-3-Phosphoethanolamine-N-[Methoxy(Polyethylene glycol)-5000] (Ammonium Salt), 1,2-Dioleoyl-3-Trimethylammonium-Propane (Chloride Salt) (DOTAP).

Possible lipids applicable for liposomes are supplied by Avanti, Polar Lipids, Inc, Alabaster, AL. Additionally, the liposome suspension may include lipid-protective agents which protect lipids against free-radical and lipid-peroxidative damage on storage. Lipophilic free-radical quenchers, such as alpha-tocopherol and water-soluble iron-specific chelators, such as ferrioxianine, are preferred.

A variety of methods are available for preparing liposomes, as described in, e.g., Szoka et al., Ann. Rev. Biophys. Bioeng. 9:467 (1980), U.S. Pat. Nos. 4, 235,871, 4,501,728 and 4,837,028. Another method produces multilamellar vesicles of heterogeneous sizes. In this method, the vesicle-forming lipids are dissolved in a suitable organic solvent or solvent system and dried under vacuum or an inert gas to form a thin lipid film. If desired, the film may be redissolved in a suitable solvent, such as tertiary butanol, and then lyophilized to form a more homogeneous lipid mixture which is in a more easily hydrated powder-like form. This film is covered with an aqueous solution of the targeted drug and the targeting component and allowed to hydrate, typically over a 15-60 minute period with agitation. The size distribution of the resulting multilamellar vesicles can be shifted toward smaller sizes by hydrating the lipids under more vigorous agitation conditions or by adding solubilizing detergents such as deoxycholate.

Micelles are formed by surfactants (molecules that contain a hydrophobic portion and one or more ionic or otherwise strongly hydrophilic groups) in aqueous solution.

Common surfactants well known to one of skill in the art can be used in the micelles of the present disclosure. Suitable surfactants include sodium laureate, sodium oleate, sodium lauryl sulfate, octaoxyethylene glycol monododecyl ether, octoxynol 9 and PLURONIC F-127 (Wyandotte Chemicals Corp.). Preferred surfactants are nonionic polyoxyethylene and polyoxypropylene detergents compatible with IV injection such as, TWEEN-80, PLURONIC F-68, n-octyl-beta-D-glucopyranoside, and the like. In addition, phospholipids, such as those described for use in the production of liposomes, may also be used for micelle formation.

In some cases, it will be advantageous to include a compound, which promotes delivery of the active substance to its target.

### Dose

By "effective amount" of GM-CSF it is meant a dose, which, when administered via pulmonary administration, achieves a concentration in the subject's airways and/or lung parenchyma which enhances pulmonary host defense.

The preparations are administered in a manner compatible with the dosage formulation, and in such amount as will be therapeutically effective. The quantity to be administered depends on the subject to be treated, including, e.g. the weight and age of the subject, the disease to be treated and the stage of disease. Suitable dosage ranges are per kilo body weight normally of the order of several hundred µg active ingredient per administration with a preferred range of from about 0.1 µg to 10000 µg per kilo body weight. Doses expected to provide an effective amount of GM-CSF comprise GM-CSF are often in the range of from 0.1 µg to 5000 µg per kilo body weight, such as in the range of from about 0.1 µg to 3000 µg per kilo body weight, and especially in the range of from about 0.1 µg to 1000 µg per kilo body weight, preferably in the range of 5 µg to 500 µg, even more about 50 µg to about 200 µg administered via inhalation once or twice daily.

Suitable daily dosage ranges are per kilo body weight per day normally of the order of several hundred µg active ingredient per day with a preferred range of from about 0.1 µg to 10000 µg per kilo body weight per day. Using monomeric forms of the compounds, the suitable dosages are often in the range of from 0.1 µg to 5000 µg per kilo body weight per day, such as in the range of from about 0.1 µg to 3000 µg per kilo body weight per day, and especially in the range of from about 0.1 µg to 1000 µg per kilo body weight per day, when based on monomeric forms having a sequence identical to sequence ID NO: 1, for functional homologues and fragments the dose is calculated based on the molecular weight of the monomeric form to the molecular weight of the homologues or fragments.

Duration of dosing will typically range from 1 day to about 4 months, such as 2 days to about 3 months, for example in the range of 1-2 days to 2 months, such as in the range of 1-2 days to 1 month.

### Medical packaging

The compounds used in the present disclosure may be administered alone or in combination with pharmaceutically acceptable carriers or excipients, in either single or multiple doses. The formulations may conveniently be presented in unit dosage form by methods known to those skilled in the art.

It is preferred that the compounds according to the present disclosure are provided in a kit. Such a kit typically contains an active compound in dosage forms for administration. A dosage form contains a sufficient amount of active compound such that a desirable effect can be obtained when administered to a subject.

Thus, it is preferred that the medical packaging comprises an amount of dosage units corresponding to the relevant dosage regimen. Accordingly, in one embodiment according to the present disclosure, the medical packaging comprises a pharmaceutical composition comprising a compound as defined above or a pharmaceutically acceptable salt thereof and pharmaceutically acceptable carriers, vehicles and/or excipients, said packaging comprising from 1 to 7 dosage units, thereby having dosage units for one or more days, or from 7 to 21 dosage units, or multiples thereof, thereby having dosage units for one week of administration or several weeks of administration.

The dosage units can be as defined above. The medical packaging may be in any suitable form for intratracheal, intrabronchial or intraalveolar administration. In a preferred embodiment according to the present disclosure the packaging is in the form of a vial, ampule, tube, blister pack, cartridge or capsule.

When the medical packaging comprises more than one dosage unit, it is preferred that the medical packaging is provided with a mechanism to adjust each administration to one dosage unit only.

Preferably, a kit contains instructions indicating the use of the dosage form to achieve a desirable affect and the amount of dosage form to be taken over a specified time period. Accordingly, in one embodiment according to the present disclosure the medical packaging comprises instructions for administering the pharmaceutical composition.

Even more preferably a freeze-dried GM-CSF preparation may be pre-packaged for example in single dose units. In an even more preferred embodiment according to the present disclosure the single dose unit is adjusted to the patient.

### Examples

### Example 1: Inhaled GM-CSF in a CF-patient

Inhaled GM-CSF was given to a CF-patient with a chronic pulmonary infection with mycobacterium abscessus since april 2000. When the infection started, the patient was 13.5 years old. Over the next 2 years, forced expiratory volume in first second (FEV₁) dropped from 80% predicted to 35% predicted.

In 2002 the patient subsequently also acquired chronic pulmonary infection with *Pseudomonas aeruginosa* and *Achromobacter xylosoxidans.* Treated with GM-CSF intra-musculary for about 1 year from 2002-2003. During this treatment pulmonary function increased to a FEV₁ of 55% predicted.

Since December 2006 the patient was treated with inhaled granulocyte macrophage colony stimulating factor (GM-CSF), in a dose of 250 mikro-gram b.i.d. every other week for twelve weeks. During this treatment, FEV₁ increased around 5-6% predicted. The patient did not experience any adverse events. Treatment with inhaled GM-CSF was restarted in September 2007 and FEV₁ has increased around 5% predicted during the first month. All infections have been treated with inhaled antibiotics continuously and with intravenous antibiotics for 2 weeks 4-6 times a year.

## Claims

1. Granulocyte-macrophage colony stimulating factor (GM-CSF), or a functional homologue thereof having at least 75% sequence identity to human GM-CSF, for use in the treatment of pulmonary diseases associated with bacterial, fungal and/or viral infection or colonization of the pulmonary system, wherein said GM-CSF, or a functional homologue thereof, is to be administered via pulmonary administration.

2. GM-CSF, or a functional homologue thereof having at least 75% sequence identity to human GM-CSF, for use according to claim 1, wherein said treatment alleviates the symptoms of and/or treats said pulmonary diseases.

3. GM-CSF, or a functional homologue thereof having at least 75% sequence identity to human GM-CSF, for use according to claim 1, wherein said treatment increases defense against infection in the pulmonary system of the host.

4. GM-CSF, or a functional homologue thereof having at least 75% sequence identity to human GM-CSF, for use according to any of the preceding claims, wherein the pulmonary diseases are selected from the group of pneumonia with bacterial, fungal and/or viral infection or colonization such as Pneumocystis carinii pneumonia, community acquired pneumonia, nosocomial pneumonia and ventilator associated pneumonia; cystic fibrosis with bacterial, fungal and/or viral infection or colonization; bronchitis with bacterial, fungal and/or viral infection or colonization; bronchiectasis with bacterial, fungal and/or viral infection or colonization; bronchiolitis with bacterial, fungal and/or viral infection or colonization including diffuse panbronchiolitis, bronchiolitis obliterans and bronchiolitis obliterans organizing pneumonia (BOOP) with bacterial, fungal and/or viral infection or colonization.

5. GM-CSF, or a functional homologue thereof having at least 75% sequence identity to human GM-CSF, for use according to any of the preceding claims, wherein an effective amount of GM-CSF is administered by intratracheal, intrabronchial or intraalveolar administration.

6. GM-CSF, or a functional homologue thereof having at least 75% sequence identity to human GM-CSF, for use according to any of the preceding claims, wherein a solution of GM-CSF is to be administered by bronchoalveolar lavage.

7. GM-CSF, or a functional homologue thereof having at least 75% sequence identity to human GM-CSF, for use according to any of the preceding claims, wherein a solution of GM-CSF is to be administered via blind tracheal washing.

8. GM-CSF, or a functional homologue thereof having at least 75% sequence identity to human GM-CSF, for use according to any of the preceding claims, wherein a nebulized solution or a suspension of GM-CSF is to be administered.

9. GM-CSF, or a functional homologue thereof having at least 75% sequence identity to human GM-CSF, for use according to any of the preceding claims, wherein an aerosol or inhaled powder form of GM-CSF is to be administered.

10. GM-CSF, or a functional homologue thereof having at least 75% sequence identity to human GM-CSF, for use according to any of the preceding claims, wherein a pegylated, liposomal or nanoparticle prepared form of GM-CSF is to be administered.

11. GM-CSF, or a functional homologue thereof having at least 75% sequence identity to human GM-CSF, for use according to any of the preceding claims, wherein GM-CSF is to be administered by direct application during bronchoscopy.

12. Use of GM-CSF, or a functional homologue thereof having at least 75% sequence identity to human GM-CSF, for the manufacture of a medicament for use in the treatment of pulmonary diseases associated with bacterial, fungal and/or viral infection or colonization of the pulmonary system, wherein said GM-CSF, or a functional homologue thereof, is to be administered via pulmonary administration.

## Patentansprüche

1. Granulozyten-Makrophagen-Koloniestimulierender Faktor (GM-CSF), oder ein funktionales Homologes davon, welches mindestens 75 % Sequenzidentität zu menschlichem GM-CSF aufweist, zur Verwendung in der Behandlung von Lungenerkrankungen mit assoziierter bakterieller, Pilz- und/oder Virusinfektion oder -Kolonisierung des pulmonalen Systems, wobei der GM-CSF, oder ein funktionales Homologes davon, über pulmonale Verabreichung zu verabreichen ist.

2. GM-CSF, oder ein funktionales Homologes davon, welches mindestens 75 % Sequenzidentität zu menschlichem GM-CSF aufweist, zur Verwendung nach Anspruch 1, wobei die Behandlung die Symptome der Lungenerkrankungen lindert und/oder die Lungenerkrankungen behandelt.

3. GM-CSF, oder ein funktionales Homologes davon, welches mindestens 75 % Sequenzidentität zu menschlichem GM-CSF aufweist, zur Verwendung nach Anspruch 1, wobei die Behandlung die Abwehr gegen Infektionen im pulmonalen System des Wirts verstärkt.

4. GM-CSF, oder ein funktionales Homologes davon, welches mindestens 75 % Sequenzidentität zu menschlichem GM-CSF aufweist, zur Verwendung nach einem der vorhergehenden Ansprüche, wobei die Lungenerkrankungen ausgewählt sind aus der Gruppe der Pneumonie mit bakterieller, Pilz- und/oder Virusinfektion oder -Kolonisierung, wie zum Beispiel Pneumocystis carinii pneumonia, ambulant erworbene Pneumonie, nosokomiale Pneumonie und mit einem Beatmungsgerät assoziierte Pneumonie; zystische Fibrose mit bakterieller, Pilz- und/oder Virusinfektion oder -Kolonisierung; Bronchitis mit bakterieller, Pilz- und/oder Virusinfektion oder -Kolonisierung; Bronchiektasie mit bakterieller, Pilz- und/oder Virusinfektion oder -Kolonisierung; Bronchiolitis mit bakterieller, Pilz- und/oder Virusinfektion oder - Kolonisierung, einschließlich diffuse Panbronchiolitis, Bronchiolitis obliterans und Bronchiolitis obliterans mit organisierender Pneumonie (BOOP) mit bakterieller, Pilz- und/oder Virusinfektion oder -Kolonisierung.

5. GM-CSF, oder ein funktionales Homologes davon, welches mindestens 75 % Sequenzidentität zu menschlichem GM-CSF aufweist, zur Verwendung nach einem der vorhergehenden Ansprüche, wobei eine effektive Menge von GM-CSF über intratracheale, intrabronchiale oder intraalveoläre Verabreichung verabreicht wird.

6. GM-CSF, oder ein funktionales Homologes davon, welches mindestens 75 % Sequenzidentität zu menschlichem GM-CSF aufweist, zur Verwendung nach einem der vorhergehenden Ansprüche, wobei eine GM-CSF-Lösung über bronchoalveoläre Lavage zu verabreichen ist.

7. GM-CSF, oder ein funktionales Homologes davon, welches mindestens 75 % Sequenzidentität zu menschlichem GM-CSF aufweist, zur Verwendung nach einem der vorhergehenden Ansprüche, wobei eine GM-CSF-Lösung über Blind-Tracheal-Waschung zu verabreichen ist.

8. GM-CSF, oder ein funktionales Homologes davon, welches mindestens 75 % Sequenzidentität zu menschlichem GM-CSF aufweist, zur Verwendung nach einem der vorhergehenden Ansprüche, wobei eine zerstäubte Lösung oder eine Suspension von GM-CSF zu verabreichen ist.

9. GM-CSF, oder ein funktionales Homologes davon, welches mindestens 75 % Sequenzidentität zu menschlichem GM-CSF aufweist, zur Verwendung nach einem der vorhergehenden Ansprüche, wobei GM-CSF als Spray oder als zu inhalierendes Pulver zu verabreichen ist.

10. GM-CSF, oder ein funktionales Homologes davon, welches mindestens 75 % Sequenzidentität zu menschlichem GM-CSF aufweist, zur Verwendung nach einem der vorhergehenden Ansprüche, wobei GM-CSF in pegylierter, liposomaler oder mit Nanopartikeln vorbereiteter Form zu verabreichen ist.

11. GM-CSF, oder ein funktionales Homologes davon, welches mindestens 75 % Sequenzidentität zu menschlichem GM-CSF aufweist, zur Verwendung nach einem der vorhergehenden Ansprüche, wobei GM-CSF über direkte Verabreichung während einer Bronchoskopie zu verabreichen ist.

12. Verwendung von GM-CSF, oder einem funktionalen Homologen davon, welches mindestens 75 % Sequenzidentität zu menschlichem GM-CSF aufweist, zur Herstellung eines Medikaments zur Verwendung in der Behandlung von Lungenerkrankungen mit assoziierter bakterieller, Pilz- und/oder Virusinfektion oder -Kolonisierung des pulmonalen Systems, wobei der GM-CSF, oder ein funktionales Homologes davon, über pulmonale Verabreichung zu verabreichen ist.

## Revendications

1. Facteur de stimulation de colonie granulocyte-macrophage (GM-CSF), ou un homologue fonctionnel de celui-ci ayant une identité de séquence d'au moins 75 % au GM-CSF humain, pour une utilisation dans le traitement de maladies pulmonaires associées à une infection ou une colonisation bactérienne, fongique et/ou virale du système pulmonaire, dans lequel ledit GM-CSF, ou un homologue fonctionnel de celui-ci, est administré par voie pulmonaire.

2. GM-CSF, ou un homologue fonctionnel de celui-ci ayant une identité de séquence d'au moins 75 % au GM-CSF humain, pour une utilisation selon la revendication 1, dans lequel ledit traitement soulage les symptômes desdites et/ou traite lesdites maladies pulmonaires.

3. GM-CSF, ou un homologue fonctionnel de celui-ci ayant une identité de séquence d'au moins 75 % au GM-CSF humain, pour une utilisation selon la revendication 1, dans lequel ledit traitement augmente la défense contre l'infection dans le système pulmonaire de l'hôte.

4. GM-CSF, ou un homologue fonctionnel de celui-ci ayant une identité de séquence d'au moins 75 % au GM-CSF humain, pour une utilisation selon l'une quelconque des revendications précédentes, dans lequel les maladies pulmonaires sont choisies dans le groupe composé de la pneumonie en présence d'une infection ou d'une colonisation bactérienne, fongique et/ou virale telle que la pneumonie à Pneumocystis carinii, la pneumonie communautaire, la pneumonie nosocomiale et la pneumonie associée au ventilateur ; la fibrose kystique en présence d'une infection ou d'une colonisation bactérienne, fongique et/ou virale; la bronchite en présence d'une infection ou d'une colonisation bactérienne, fongique et/ou virale; la bronchiectasie, en présence d'une infection ou d'une colonisation bactérienne, fongique et/ou virale; la bronchiolite en présence d'une infection ou d'une colonisation bactérienne, fongique et/ou virale, y compris la panbronchiolite diffuse, la bronchiolite oblitérante et la bronchiolite oblitérante avec organisation pneumonique (BOOP) en présence d'une infection ou d'une colonisation bactérienne, fongique et/ou virale.

5. GM-CSF, ou un homologue fonctionnel de celui-ci ayant une identité de séquence d'au moins 75 % au GM-CSF humain, pour une utilisation selon l'une quelconque des revendications précédentes, dans lequel une quantité efficace de GM-CSF est administrée par voie intra-trachéale, intra-bronchique ou intra-alvéolaire.

6. GM-CSF, ou un homologue fonctionnel de celui-ci ayant une identité de séquence d'au moins 75 % au GM-CSF humain, pour une utilisation selon l'une quelconque des revendications précédentes, dans lequel une solution de GM-CSF doit être administrée par lavage broncho-alvéolaire.

7. GM-CSF, ou un homologue fonctionnel de celui-ci ayant une identité de séquence d'au moins 75 % au GM-CSF humain, pour une utilisation selon l'une quelconque des revendications précédentes, dans lequel une solution de GM-CSF doit être administrée par lavage trachéal à l'aveugle.

8. GM-CSF, ou un homologue fonctionnel de celui-ci ayant une identité de séquence d'au moins 75 % au GM-CSF humain, pour une utilisation selon l'une quelconque des revendications précédentes, dans lequel une solution nébulisée ou une suspension de GM-CSF doit être administrée.

9. GM-CSF, ou un homologue fonctionnel de celui-ci ayant une identité de séquence d'au moins 75 % au GM-CSF humain, pour une utilisation selon l'une quelconque des revendications précédentes, dans lequel un aérosol ou une poudre d'inhalation de GM-CSF doit être administré.

10. GM-CSF, ou un homologue fonctionnel de celui-ci ayant une identité de séquence d'au moins 75 % au GM-CSF humain, pour une utilisation selon l'une quelconque des revendications précédentes, dans lequel une forme pégylée, liposomale ou nanoparticulaire préparée de GM-CSF doit être administrée.

11. GM-CSF, ou un homologue fonctionnel de celui-ci ayant une identité de séquence d'au moins 75 % au GM-CSF humain, pour une utilisation selon l'une quelconque des revendications précédentes, dans lequel le GM-CSF doit être administré par application directe au cours d'une bronchoscopie.

12. Utilisation du GM-CSF, ou d'un homologue fonctionnel de celui-ci ayant une identité de séquence d'au moins 75 % au GM-CSF humain, pour la préparation d'un médicament pour une utilisation dans le traitement de maladies pulmonaires associées à une infection ou une colonisation bactérienne, fongique et/ou virale du système pulmonaire, dans laquelle le GM-CSF, ou un homologue fonctionnel de celui-ci, doit être administré par voie pulmonaire.
